# EUROPÄISCHE PATENTSCHRIFT

(11) **EP 2 393 561 B1**
(45) Veröffentlichungstag und Bekanntmachung des Hinweises auf die Patenterteilung: **02.10.2013**
(21) Anmeldenummer: 10710172.7
(22) Anmeldetag: 04.02.2010
(51) Int. Cl.: A63B 23/18

(54) **Tragbarer Atemtakter**
Portable breath regulator
Indicateur de cycle respiratoire portatif

(30) Priorität: 06.02.2009 DE 102009007934
(43) Veröffentlichungstag der Anmeldung: 14.12.2011
(73) Patentinhaber: Fehrl, Heidrun, 38116 Braunschweig (DE)
(72) Erfinder: Fehrl, Heidrun, 38116 Braunschweig (DE)
(74) Vertreter: Lins, Edgar
(86) Internationale Anmeldenummer: PCT/DE2010/000134
(87) Internationale Veröffentlichungsnummer: WO 2010/088895

(56) Entgegenhaltungen:
- WO-A1-2007/133121
- DE-A1- 10 227 760
- US-A- 4 711 585
- US-A1- 2003 065 272

## Beschreibung

Die Erfindung betrifft einen Atemtakter, mit dem ein Benutzer in der Lage ist, eine gesunde Atmung zu erwerben, in akuten Stresssituationen seine Körperfunktionen (Puls, Herzschlag, vegetatives Nervensystem) zu kontrollieren und die Stressbelastung durch Cortisol etc. sofort zu reduzieren.

Ein Atemtakter gibt ein von einem Benutzer wahrnehmbares Signal aus, das ihm die Einatmungsphasen, Ausatmungsphasen und ggf. Pausenphasen vorgibt. An diesen Phasen richtet der Benutzer seine Atmung aus.

Eine gesunde Atmung ist wichtig für eine Vorbeugung und Linderung von Stresserkrankungen. Physische und psychische Stresserkrankungen nehmen insbesondere in Industriegesellschaften stetig zu. Stress ist Auslöser zahlreicher gesundheitlicher Probleme mit den daraus resultierenden betriebswirtschaftlichen und gesamtgesellschaftlichen Kosten.

Stress kann für viele Folgeerkrankungen verantwortlich sein, beispielsweise für Bluthochdruck, Rückenschmerzen, Herz-Kreislauf-Erkrankungen, Angina Pectoris, Herzinfarkt und Schlaganfall. Darüber hinaus verursacht Stress Konzentrations- und Leistungsstörungen, chronische Erschöpfung, Angsterkrankungen, Panikattacken und Depressionen. Zudem steht Stress im Verdacht, die Gefahr von Tumorerkrankungen zu erhöhen. Stress kann auch chronisch werden und die Gesundheit dauerhaft beeinträchtigen und gefährden.

Aus der DE 693 19 742 T2 ist ein Atemregler bekannt, mit dem Atmungsregelmäßigkeitsstörungen behandelt werden. Der Atemregler bestimmt das Verhältnis zwischen der Einatmungszeit und der Ausatmungszeit in einem Atmungszyklus. In Abhängigkeit dieses Verhältnisses wird ein von dem Benutzer wahrnehmbares akustisches Signal ausgegeben, an dem der Benutzer seine Atmung ausrichtet. Wenn der Atemregler durch Auswertung des Verhältnisses feststellt, dass die Atmung des Benutzers wieder mit einem vorgegebenen gesunden Atmungsmuster übereinstimmt, schaltet er sich selbsttätig ab. Nachteilig ist, dass der Atemregler einen komplizierten technischen Aufbau aufweist und damit kostspielig in der Herstellung ist. Zudem ist er nicht einfach zu bedienen. Ein weiterer Nachteil besteht darin, dass er auffällig und für Dritte wahrnehmbar ist.

Aus der DE 102 27 760 A1 ist ein Signalgeber zur Atemregulierung bekannt, mit dem insbesondere Yoga-Übungen, die der Regulierung und Rhythmisierung des Atems dienen sollen, durchführbar sind. Der dort beschriebene Atemtakter lässt sich so einstellen, dass die Länge der einzelnen Atemphasen zum einen frei einstellbar ist und zum anderen über einen ebenfalls frei einstellbaren Anpassungswert über den Verlauf einer jeweiligen Übung veränderbar sind.

Aus der WO 2007/133121 ist ein Atemkontrollverfahren zur Einwirkung auf einen Organismus bekannt, bei dem Atemübungen der Yogis verwendet werden sollen, wodurch eine Hypoxie erzeugt werden soll. Dabei misst ein Gerät die Herzkontraktionsfrequenz der atmenden Person und ermittelt aus dieser die jeweilige Länge der einzelnen Phasen der Atmung.

Aus der US 4,711,585 ist ein Gerät bekannt, mit dem die Atmung werdender Mütter beeinflusst werden soll, um so eine leichtere Geburt hervorzurufen. Dabei wird in verschiedenen Stadien der Geburt jeweils ein eigener Atemrhythmus vorgegeben, der fest im Gerät eingestellt ist.

Der Erfindung liegt die Aufgabe zu Grunde, einen Atemtakter mit weiter verbesserter Funktionalität anzugeben.

Die Aufgabe wird durch einen Atemtakter mit den Merkmalen des unabhängigen Anspruchs gelöst.

Erfindungsgemäß ist die Steuereinheit des Atemtakters dazu eingerichtet, die Dauer der Einatmungsphase und/oder der Ausatmungsphase während einer Benutzungsperiode des Atemtakters automatisch zu verändern. Hierdurch kann der Atemrhythmus situationsbedingt an die jeweiligen Bedürfnisse angepasst werden. Beispielsweise ist es möglich, von einem bestimmten Start-Atemrhythmus hin zu einem Entspannungs-Atemrhythmus überzugehen, bei dem die Dauer der Einatmungsphase und der Ausatmungsphase gegenüber dem Start-Atemrhythmus verlängert sind. Die Benutzungsperiode erstreckt sich typischerweise über einige Minuten, beispielsweise 10 Minuten. Zudem ist erfindungsgemäß ein Bediengerät vorgesehen, das nur bestimmte voreingstellte Kombinationen der Dauer der Einatmungsphase und der Ausatmungsphase erlaubt.

Die Übertragung des Signals auf den Körper eines Benutzers kann direkt erfolgen, z. B. über die Haut oder das Gehör, oder indirekt, z. B. über Bekleidung oder andere mit dem Körper verbundene Gegenstände.

Durch die Erfindung wird ein einfach herzustellendes, kostengünstiges und auch von Kindern - z.B. mit ADHS (Hyperaktivität) - leicht bedienbares Gerät bereitgestellt, mit dem ein Benutzer eine gesunde Atmung erlernen kann. Durch die Erfindung wird außerdem ein Gerät bereitgestellt, das unauffällig getragen werden kann.

Der erfindungsgemäße Atemtakter umfasst einfache und kostengünstige Komponenten, wie zum Beispiel eine Signaleinheit zum Übertragen einer Vibration, eines Stromimpulses oder einer Temperaturänderung. Diese Signale können einzeln oder in beliebiger Kombination übertragbar sein.

Die Steuereinheit generiert ein periodisches Ansteuersignal, mit dem die Signaleinheit angesteuert wird und ein entsprechendes periodisches Signal ausgibt, das von einem Benutzer wahrnehmbar ist. Die Steuereinheit kann hierzu einen geeignet programmierten Mikrocontroller umfassen. Die Daten für das Ansteuersignal entnimmt die Steuereinheit beispielsweise einem geeigneten Speicher, der in dem Atemtakter integriert ist.

Die unterschiedlichen Phasen (Einatmungs-, Ausatmungs- und ggf. Pausenphase) sind von dem Benutzer unterschiedlich wahrnehmbar, damit er sie auseinander halten kann. Dies ist wichtig, da er seine Atmung an diesen Phasen ausrichten muss, um den gewünschten Zweck, eine gesündere Atmung, zu erreichen. Die Erfindung umfasst auch den Fall, dass eine Phase dadurch gekennzeichnet ist, dass kein positiv wahrnehmbares Signal abgegeben wird, wenn die zeitlich unmittelbar angrenzenden Phasen positive Signale umfassen. Entscheidend ist die Erkennbarkeit der einzelnen Phasen für den Benutzer. Positiv wahrnehmbare Signale können gleichmäßig oder ungleichmäßig sein.

Der erfindungsgemäße Atemtakter verzichtet im Gegensatz zu dem bekannten Atemregler auf eine Regeleinrichtung, die mit komplizierten elektronischen Schaltungen realisiert ist und geeignete Sensoren zum Detektieren von physischen Zuständen des Benutzers benötigt. Eine Regeleinrichtung, einschließlich der erforderlichen Sensoren, ist kostspielig. Zudem macht sie die Bedienung des Atemreglers kompliziert, was Kinder und ältere Menschen sowie Menschen mit geringen intellektuellen Fähigkeiten in der Nutzung einschränken würde.

Der erfindungsgemäße Atemtakter ist jedoch in der Lage, die Atmung des Benutzers auf Dauer genauso positiv zu beeinflussen, wie die komplizierten bekannten Geräte. Es hat sich nämlich gezeigt, dass das Ergebnis der Atemübung davon abhängt, dass die Atemübung regelmäßig und von angemessener täglicher Dauer durchgeführt wird.

Der erfindungsgemäße Atemtakter ist tragbar und erfordert keine visuelle Kontrolle. Der Benutzer braucht also nicht zu liegen oder seine visuelle. Aufmerksamkeit dem Atemtakter zu widmen. Es kann deshalb nahezu überall eingesetzt werden, beispielsweise beim Joggen, beim Autofahren, vor dem Halten von Vorträgen, in Gefahrensituationen (Feuerwehr, Rettungspersonal, Taucher etc.) oder generell in oder vor Situationen, die Konzentration und Aufmerksamkeit erfordern.

Der erfindungsgemäße Atemtakter wirkt sich positiv auf die Gesundheit des Benutzers aus, insbesondere durch eine Erhöhung der Herzratenvariabilität (HRV: Schwankungen der Herzfrequenz von Schlag zu Schlag) und Herzkohärenz (Zustand der Synchronisation von Atmung, Herzschlag und Blutdruck). Der HRV-Wert ist ein Parameter, der physiologische Zustände anzeigt. Mit ihm werden Stressbelastungen und Zustände des Organsystems gemessen. Der gemessene HRV-Wert ist ein zuverlässiger Indikator für unterschiedliche Störungen. Die Erhöhung der Anpassungsleistung des Herzens (Herzkohärenz) durch Einsatz des Atemtakters führt zu Prävention und Rückgang von riskanten Organzuständen. Zu nennen sind etwa:
- Stressbedingt hoher Blutdruck wird ohne medikamente gesenkt
- Ein- und Durchschlafstörungen werden verringert
- Ängste und Panikstörungen können besser kontrolliert werden
- Herz- und Kreislauf werden wieder ins Gleichgewicht gebracht (bei erlittenem Herzinfarkt besteht bei einer hohen HRV eine neunmal bessere Chance, keinen zweiten Herzinfarkt zu erleiden)
- Bei kurz dauernder Diabetes wird die Zerstörung des Vagusnervs hinausgezögert
- Die Produktion schädlicher Stresshormone wird gedrosselt
- Die DHEA-Werte (DHEA: Dehydroepiandrosteron, Zellerneuerungshormon) erhöhen sich bei einer täglichen Atemübung von insgesamt dreißig Minuten nach achtundzwanzig Tagen bereits um 100% (laut vorliegenden Studien)
- Die Immunkräfte werden gestärkt
- Konzentration und Leistungsfähigkeit werden gesichert
- Die generelle Stressresistenz wird erhöht
- Zusammen mit Stretching verdoppelt sich die HRV in nur achtundzwanzig Tagen (selbst bei seit einem Jahr bestehendem Muskel- und Kreislauftraining)
- Die Alterung wichtiger Organsysteme wird durch eine hohe HRV gehemmt

Für die Stromversorgung des erfindungsgemäßen Atemtakters wird vorzugsweise eine Batterie verwendet, die in dem Atemtakter austauschbar integriert ist. Geeignete Batterien sind beispielsweise von Armbanduhren bekannt. Der Atemtakter kann jedoch auch mit einem Akkumulator versehen sein, der über ein anschließbares Ladekabel oder eine Solarzelle aufladbar ist. Es ist auch möglich, dass der Akkumulator über einen automatischen Aufzug aufgeladen wird. Hierzu weist der Atemtakter beispielsweise ein bewegliches Teil auf (z.B. eine in einer Führungsnut umlaufende Kugel), das über Bewegungen des Atemtakters in Bewegungen versetzt wird und über einen kleinen Generator den Akkumulator auflädt, indem dessen kinetische Energie in elektrische Energie umgewandelt wird.

Vorteilhaft ist es, wenn der Atemtakter ein Befestigungsmittel aufweist, um ihn am Körper des Benutzers zu befestigen. Geeignete Befestigungsmittel sind beispielsweise ein Brustband, Bauchgurt, Armband, Headset, eine anatomisch oder nicht-anatomisch geformte Ohrklammer, eine Klettvorrichtung oder eine Ohrkapsel. Es ist jedoch auch möglich, dass der Atemtakter lose in einer Tasche getragen wird. Dabei ist darauf zu achten, dass die Signale vom Benutzer wahrgenommen werden können. Der Atemtakter lässt sich auch in anderes Gerät integrieren, beispielsweise in ein Handy, ein Blutdruckmessgerät, ein Oxymeter.

In einer Ausführungsform ist erfindungsgemäß vorgesehen, dass das Signal zusätzlich ein akustisches und/oder optisches Signal umfasst. Durch das zusätzliche akustische und/oder optische Signal wird die Wahrnehmbarkeit des Signals verbessert oder für mehrere Personen gleichzeitig sichtbar gemacht. Als akustisches Signal kommt im Prinzip jeder Ton in Betracht, bevorzugt ein Ton eines Instruments, eine Stimme oder ein natürliches Geräusch.

Vorteilhaft ist es, wenn das Signal der Signaleinheit einstellbar ist. Beispielsweise lässt sich die Lautstärke des akustischen Signals durch einen an dem Atemtakter vorgesehenen Lautstärkeregler verändern. Im Falle eines Vibrationssignals ist vorteilhaft die Stärke der Vibration einstellbar.

Zweckmäßig ist es, wenn die Periodendauer, die Dauer der Einatmungsphase, die Dauer der Ausatmungsphase und die Dauer einer optionalen Pausenphase für den Beginn der Benutzungsperiode einstellbar sind. Dies ermöglicht eine Anpassung an verschiedene Benutzer mit unterschiedlichen Atmungsrhythmen, beispielsweise für die verschiedenen Personen einer Familie. So können mehrere Personen ein einziges Gerät benutzen. Dies erhöht die Wirtschaftlichkeit des erfindungsgemäßen Atemtakters. Zweckmäßig weist der Atemtakter einen Speicher mit Speicherplätzen für individuelle Einstellungen des Atemtakters (z. B. Abstände der Atemübungen, Vibrationsstärke, Lautstärke, Temperatur) auf. Die einzelnen Einstellungen lassen sich über ein geeignetes Eingabeelement (z. B. einen Knopf) abrufen.

Es hat sich als vorteilhaft herausgestellt, wenn die Dauer der Einatmungsphase nicht länger ist als die Dauer der Ausatmungsphase, insbesondere wenn die Einatmungsphase kürzer - vorzugsweise etwa 1 bis 2 Sekunden kürzer - ist als die Dauer der Ausatmungsphase bzw. wenn pro Minute bevorzugt 5 bis 10 (je nach Gewicht und Bedarf des Anwenders) komplette Atemzyklen stattfinden, die das physiologische Gleichgewicht optimal regulieren, wie aus neuesten Forschungsergebnissen hervorgeht. Aus diesem Grunde ist es zweckmäßig, wenn der erfindungsgemäße Atemtakter entsprechend ausgebildet ist. In einer vorteilhaften Ausführungsform weist die Einatmungsphase eine Dauer von 1 bis 10 Sekunden und die Ausatmungsphase eine Dauer von 2 bis 20 Sekunden auf. Optional kann nach der Ausatmungsphase eine Pausenphase vorgesehen sein. Diese hat vorteilhaft eine Dauer von 0,5 bis 2 Sekunden, vorzugsweise von etwa 1 Sekunde.

Gemäß einer vorteilhaften Weiterbildung der Erfindung ist der Atemtakter dafür eingerichtet, nur auf gesundheitsfördernde Einstellungen der Einatmungsphase und der Ausatmungsphase einstellbar zu sein. Vorteilhaft sind beispielsweise in einer Betriebssoftware des Atemtakters Abfragen vorgesehen, die eine Einstellung einer Einatmungsphase, die länger als die Ausatmungsphase ist, verhindern. Gemäß einer weiteren vorteilhaften Weiterbildung der Erfindung weist der Atemtakter eine Auswahleinrichtung auf, mittels der medizinisch sinnvolle Zeiten für die Einatmungsphase und die Ausatmungsphase in Abhängigkeit von der Körpergröße und dem Körpergewicht der benutzenden Person ausgewählt werden. Gemäß einer vorteilhaften Weiterbildung sind solche von Größe und Gewicht abhängigen Einstellungen in dem Atemtakter speicherbar. Vorteilhaft ist eine Mehrzahl von Speicherplätzen vorgesehen, die es einer Mehrzahl von Benutzern des Atemtakters erlauben, jeweils auf einfache und schnelle Weise eine persönliche Einstellung zu speichern und abzurufen.

Gemäß einer vorteilhaften Weiterbildung der Erfindung sind bei dem Atemtakter eine oder mehrere Benutzunsperioden einstellbar. Als Benutzungsperiode wird in diesem Zusammenhang ein Einschaltzyklus des Atemtakters bzw. ein Nutzungszyklus eines Benutzers verstanden. Der Benutzer kann somit aus einer von mehreren Perioden eine geeignete Einstellung auswählen. Die Perioden können auch voreingestellt sein. Gemäß einer vorteilhaften Weiterbildung der Erfindung weisen die Perioden unterschiedliche Atemrhythmen auf. Unter einem Atemrhythmus wird das Verhältnis zwischen Einatmungsphase und Ausatmungsphase verstanden. Gemäß einer weiteren vorteilhaften Weiterbildung der Erfindung weist eine Periode mehrere unterschiedliche Atemrhythmen auf. Vorteilhaft sind mehrere gleiche oder unterschiedliche Perioden mit mehreren unterschiedlichen Atemrhythmen pro Periode einstellbar. Hierdurch ist es möglich, einen Atemrhythmus einzustellen, der erfindungsgemäß innerhalb einer Benutzungsperiode unterschiedlich ist, d. h. sich während der Benutzungsperiode verändert. Hierbei ist es vorteilhaft, zu Beginn der Benutzungsperiode des Atemtakters mit kürzeren Zeitdauern der Einatmungsphase und der Ausatmungsphase zu beginnen und diese im Verlauf der Nutzung zu längeren Zeitdauern hin zu verändern.

Gemäß einer vorteilhaften Weiterbildung der Erfindung kann der Atemtakter auch baulich vereint oder integriert mit anderen medizintechnischen Geräten bzw. mit Geräten, die Körperzustände messen, ausgeführt sein. Als Beispiele seien hier Blutdruckmessgeräte, Oxymeter oder Pulsmesser genannt. Vorteilhaft ist auch eine Integration bzw. bauliche Vereinigung des Atemtakters mit Spielzeug, wie etwa Plüschtieren oder Robotern, oder therapeutischen künstlichen Tieren, insbesondere solchen, die dazu ausbildet sind, Körperzustände wie z.B. Herzschlag oder Blutdruck zu messen, wie z.B. einer z. Zt. auf dem Markt befindlichen Robbe. Vorteilhaft können hierbei in dem Spielzeug oder dem therapeutischen Tier bereits vorgesehene Ausgabemittel, wie Tonsignalgeber oder Vibrationsgeber, für die Vorgabe eines gesunden Atemtakts mit verwendet werden. Der in ein solches Gerät integrierte oder damit baulich vereinte Atemtakter kann dabei relativ einfach aufgebaut sein und die von dem Wirtsgerät bereitgestellte Infrastruktur nutzen.

Gemäß einer vorteilhaften Weiterbildung der Erfindung ist der Atemtakter dazu eingerichtet, von eigenen Sensoren oder über eine Schnittstelle zu einem medizintechnischen Gerät Daten zu empfangen, die wenigstens einen Körperzustand des Benutzers wie z. B. Blutdruck oder Pulsfrequenz angeben. Gemäß einer vorteilhaften Weiterbildung ist der Atemtakter dazu eingerichtet, den Atemrhythmus in Abhängigkeit der empfangenen, den Körperzustand wiedergegebenen Daten zu verändern. Beispielsweise kann der Atemrhythmus abhängig von einer Pulsmessung verändert werden. Beispielsweise kann der Atemrhythmus bei relativ hoher Pulsfrequenz auf einen langsameren Atemrhythmus heruntergefahren werden. Hierdurch kann von einem Anfangsrhythmus zu einem langsameren Entspannungsrhythmus übergegangen werden.

Gemäß einer vorteilhaften Weiterbildung der Erfindung kann der Atemtakter auch als Computerprogrammprodukt ausgebildet sein, beispielsweise in Form eines Softwaremoduls für ein Mobiltelefon (Handy) oder eines der zuvor genannten Geräte, mit denen der Atemtakter baulich vereint oder integriert ausgebildet sein kann. Es ist bekannt, dass moderne Mobiltelefone durch zusätzliche Softwaremodule in ihren Funktionen erweiterbar sind, d. h. die Mobiltelefone sind hierdurch programmierbar. Solche Mobiltelefone weisen zudem in der Regel geeignete Ausgabemittel zur Ausgabe des auf den Körper des Benutzers zu übertragenden Signals eines Atemtakters auf, beispielsweise Vibrationselemente für einen Vibrationsalarm. Auf diese Weise kann ein handelsübliches Mobiltelefon durch Hinzuladen eines die Erfindung beinhaltenden Softwaremoduls um die Funktionalität eines Atemtakters erweitert werden. Vorteilhaft kann das Softwaremodul beispielsweise über das Mobiltelefonnetz oder das Internet zum Herunterladen angeboten werden. Hierdurch wird sozusagen das Mobiltelefone oder eines der zuvor genannten Geräte zu einem Atemtakter.

Gemäß einer weiteren Weiterbildung der Erfindung kann der Atemtakter beispielsweise über einen Computer extern mit gewünschten Atemrhythmen programmiert bzw. parametriert werden. Der Atemtakter weist hierfür vorteilhaft eine Schnittstelle zu einem Computer auf, beispielsweise eine USB-Schnittstelle oder eine drahtlose Schnittstelle wie Bluetooth oder Infrarot.

Gemäß einer vorteilhaften Weiterbildung der Erfindung kann über die Schnittstelle eine Geräte-Identifikation des Atemtakters ausgelesen werden und hierdurch in dem Atemtakter gespeicherte Werte, beispielsweise für einen bevorzugten Atemrhythmus, einem Benutzer zugeordnet werden. Dies hat den Vorteil, dass bei einer Mehrfachnutzung des Atemtakters durch eine Vielzahl von Benutzern, beispielsweise bei einer Verwendung in Rehabilitationseinrichtungen, eine automatisierte Verwaltung der Benutzerdaten für die Atemtakter auf einem Computer durchgeführt werden kann.

Gemäß einer vorteilhaften Weiterbildung der Erfindung weist der Atemtakter Erfassungsmittel für die Einschaltdauer bzw. die Benutzungsdauer durch einen Benutzer und/oder die benutzten Atemrhythmen auf. Hierdurch ist es möglich, die genannten Daten mit zu protokollieren und in einem Speicher des Atemtakters für eine spätere Auswertung für Diagnosezwecke abzuspeichern. Gemäß einer vorteilhaften Weiterbildung der Erfindung ist vorgesehen, dass die Signaleinheit einen Lautsprecher, eine Leuchtdiode, ein Temperaturelement, ein Stromimpulselement und/oder ein Vibrationselement aufweist. Mit diesen Elementen können die Signale auf einfache Art und Weise für den Benutzer wahrnehmbar übertragen werden. Der Lautsprecher ist vorzugsweise am Ohr vorgesehen, beispielsweise an einer Ohrklammer. Die Leuchtdiode ist vorzugsweise zum Tragen dicht an den Augen vorgesehen, beispielsweise an einer Brille bzw. einem Brillengestell. Das Temperaturelement ist vorzugsweise als isoliertes oder unisoliertes Metallelement, beispielsweise ein Metallstreifen, zum Tragen auf der Haut vorgesehen. Das Vibrationselement ist vorzugsweise als Exzenter zum Tragen auf der Haut oder am Körper vorgesehen. Es ist jedoch auch möglich, das Vibrationselement als Lautsprecher mit einer Lautsprechermembran auszubilden, die in Schwingungen mit niedrigen Frequenzen versetzt wird. Diese niederfrequenten Schwingungen sind als Vibration haptisch wahrnehmbar. Das Vibrationselement kann auch in der Hosentasche etc. getragen werden, wenn die Vibration ausreichend stark ist bzw. sich ausreichend stark einstellen lässt.

In einer bevorzugten Ausführungsform ist vorgesehen, dass das Signal während der Einatmungsphase eine Vibration zunehmender Intensität und/oder zunehmender Frequenz aufweist, wobei die Zunahme vorzugsweise gleichmäßig erfolgt. Zweckmäßig ist es in diesem Fall (aber nicht nur in diesem Fall), wenn das Signal während der Ausatmungsphase eine Vibration abnehmender Intensität und/oder abnehmender Frequenz aufweist, wobei die Abnahme vorzugsweise gleichmäßig erfolgt. Es hat sich nämlich gezeigt, dass die Menschen ihre Atmung gerne an Signalen ausrichten, die sich gleichmäßig verändern. Selbstverständlich kann die Vibration während der Ausatmungsphase auch eine zunehmende Intensität und/oder Frequenz aufweisen.

Ist das Signal ein Stromimpuls bzw. eine Stromimpulsfolge, so kann es in einer Phase mit positivem Signal gleichmäßig, zunehmend oder abnehmend gebildet sein.

Eine weitere Ausführungsform mit sich verändernden Signalen erhält man, wenn das Signal während der Einatmungsphase eine sich räumlich fortpflanzende oder ausbreitende Vibration und während der Ausatmungsphase eine sich entgegengesetzt zu der Einatmungsphase verhaltende Vibration aufweist. Zweckmäßig erfolgt die Veränderung bei dieser Ausführungsform gleichmäßig. Mit räumlich fortlaufend ist gemeint, dass die Fläche der Vibration konstant bleibt, sich jedoch der Ort (etwa auf der Haut) der Vibration ändert. Bei der sich ausbreitenden Vibration ändert sich nicht nur der Ort (ein neuer Ort kommt hinzu), sondern auch die Fläche der Vibration, beispielsweise eine sich kreisflächenförmig ausbreitende Vibration. In der Ausatmungsphase zieht sich die Kreisfläche der Vibration bei der vorstehend genannten Ausführungsform wieder zusammen (kleiner werdende Kreise).

Mit einem Temperaturelement lässt sich ein veränderndes Signal realisieren, wenn das Signal während der Einatmungsphase eine zunehmende Temperatur und während der Ausatmungsphase eine abnehmende Temperatur aufweist oder umgekehrt. Vorzugsweise erfolgt die Veränderung der Temperatur gleichmäßig.

Es hat sich herausgestellt, dass gute gesundheitliche Fortschritte erzielt werden, wenn die Atmung regelmäßig trainiert wird. Aus diesem Grunde ist erfindungsgemäß vorgesehen, dass die Steuereinheit ausgebildet ist, zu überwachen, ob eine vorgegebene Benutzungsdauer des Atemtakters in einer vorgegebenen Zeitspanne erreicht wird. Beispielsweise beträgt die vorgegebene Benutzungsdauer dreißig Minuten und die vorgegebene Zeitspanne vierundzwanzig Stunden. Zweckmäßig wird noch ein Startzeitpunkt festgelegt, beispielsweise eine bestimmte Uhrzeit. Hat der Benutzer seine Benutzungsdauer für die Zeitspanne noch nicht erfüllt, kann vorgesehen sein, dass der Atemtakter eine Warnung, beispielsweise als akustischen Ton oder optisches Signal, ausgibt. Vorzugsweise erfolgt die Warnung so rechtzeitig, dass die Benutzungsdauer noch erreicht werden kann. Die Warnung kann auch mehrmals und mit zunehmender Intensität erfolgen. Der Atemtakter kann auch so ausgebildet sein, dass er voreingestellte zeitliche Sequenzen der Benutzungsperioden zur Verfügung stellt, beispielsweise Sequenzen von 5 Minuten bis zu einer Stunde.

Der Atemtakter kann auch ohne zeitliche Begrenzung aktiviert werden. Dies ist in akuten Stresssituationen, bei Schlaflosigkeit etc. sinnvoll.

In einer besonderen Ausführungsform ist der erfindungsgemäße Atemtakter wasserdicht. So kann er auch draußen bei Regen oder im Wasser benutzt werden. Für die Benutzung des erfindungsgemäßen Atemtakters durch Taucher ist wichtig, dass der Atemtakter ausreichend stabil ist, um den auftretenden Drücken im Wasser standzuhalten. Als Grundregel gilt, dass der Druck um etwa 1 bar je 10 m Wassertiefe zunimmt. So muss der Atemtakter für Tauchgänge bis zu 20 m Wassertiefe einen Druck von mindestens 3 bar standhalten, bei 30 m Wassertiefe bereits 4 bar. Bei den für den Atemtakter verwendeten Materialien, beispielsweise für ein Gehäuse oder eine Dichtung, ist zweckmäßig zu berücksichtigen, ob der Atemtakter in Süßwasser- oder Salzwasser-Gewässern eingesetzt werden soll.

Die Erfindung wird anhand des in der folgenden Figur dargestellten Ausführungsbeispiels näher erläutert. Es zeigt
- Figur 1: eine Ausführungsform des erfindungsgemäßen Atemtakters,
- Figur 2: eine perspektivische Draufsicht auf eine weitere Ausführungsform des erfindungsgemäßen Atemtakters und
- Figur 3: eine perspektivische Rückansicht des Atemtakters aus Figur 2 und
- Figur 4: eine perspektivische Ansicht einer weiteren Ausführungsform des Atemtakers und
- Figur 5: beispielhafte Zeitverläufe von Atemrhythmen.

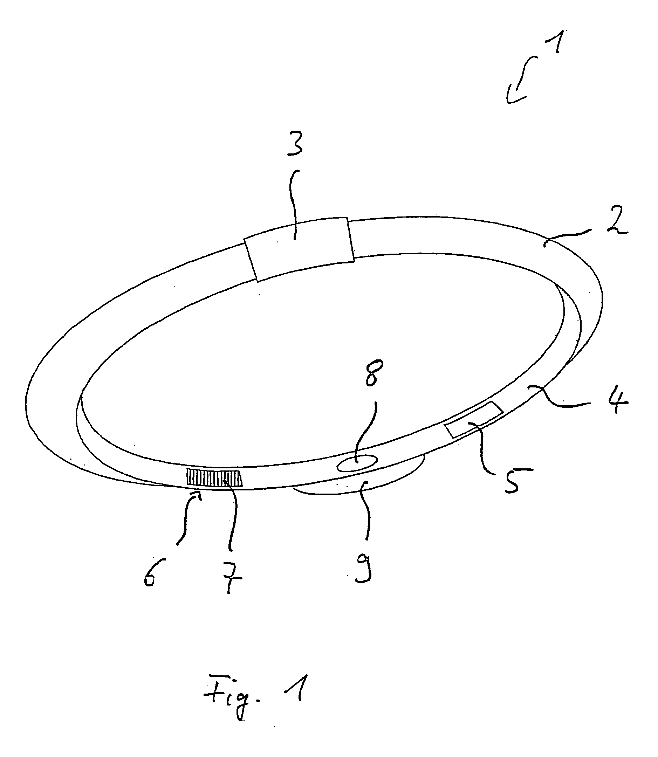

In Figur 1 ist ein tragbarer Atemtakter 1 dargestellt.

Der Atemtakter 1 umfasst ein Armband 2. Das Armband 2 lässt sich an einem Verschluss 3 öffnen und schließen.

An der Innenseite 4 (der einem Arm zugewandten Seite) des Armbandes 2 ist ein Vibrationselement 5 angebracht. Das Vibrationselement 5 wird von einer nicht dargestellten Steuereinheit mit einer Folge von Ansteuersignalen derart angesteuert, dass eine Folge von Einatmungs-, Ausatmungs- und Pausenphasen gebildet wird. Die Einatmungsphase hat eine Dauer von vier Sekunden, die Ausatmungsphase von sechs Sekunden. Eine Pausenphase ist in diesem Beispiel nicht vorgesehen.

Die Steuereinheit wird über einen nicht dargestellten Ein/Ausschalter ein- bzw. ausgeschaltet. Der Ein/Ausschalter ist an dem Armband 2 angeordnet.

An der Innenseite 4 ist ein Temperaturelement 6 angebracht. Das Temperaturelement 6 weist eine Mehrzahl im Wesentlichen paralleler Drähte 7 auf. Das Temperaturelement 6 ist mit der Steuereinheit verbunden. Die vorstehende Folge von Einatmungs-, Ausatmungs- und Pausenphasen wird in Form von unterschiedlichen Temperaturen des Temperaturelements 6 gebildet. Die Drähte sind aus Kupfer hergestellt, um eine ausreichend schnelle Temperaturänderung zu ermöglichen. Weitere geeignete Materialien für die Drähte sind Silber und Gold.

An der Innenseite 4 ist ein Pulsmesser 8 angebracht, der den Puls des Benutzers misst. Über eine LED 9 (Light Emission Diode) wird der Pulsschlag optisch angezeigt. Der gemessene Puls hat jedoch keinen Einfluss auf die Signalfolge. Es ist möglich, weitere Sensoren für physiologische Körperwerte vorzusehen, etwa für den Blutdruck oder das Erreichen der optimalen HRV bzw. Herzkohärenz, die ebenfalls keinen Einfluss auf die Signalfolge haben.

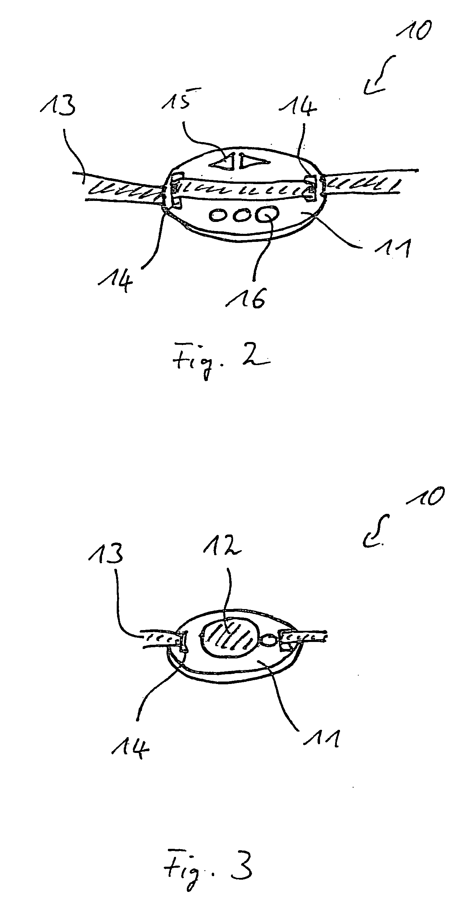

Figur 2 zeigt eine weitere Ausführungsform eines erfindungsgemäßen Atemtakters 10.

Der Atemtakter 10 umfasst ein Gehäuse 11, in das eine Signaleinheit 12 und eine nicht dargestellte Steuereinheit integriert sind.

Der Atemtakter 10 umfasst ferner ein Brustband 13 als Befestigungsmittel.

Das Brustband 13 ist durch zwei Langlöcher 14 durchgezogen, die an dem Gehäuse 11 ausgebildet sind. Durch diese Verbindung des Gehäuses 11 und des Brustbandes 13 ist es möglich, das Gehäuse 11 relativ zum Brustband 13 zu verschieben.

Der Atemtakter 10 weist Bedienelemente 15, 16 auf.

Die Bedienelemente 15 dienen der Einstellung der Stärke des Signals der Signaleinheit 12.

Mit den Bedienelementen 16 lassen sich weitere Einstellungen vornehmen, beispielsweise die Auswahl von Personeneinstellungen oder Signaleinstellungen.

Gemäß einer vorteilhaften Weiterbildung der Erfindung ist vorgesehen, dass der tragbare Atemtakter eine Signaleinheit 5, 6 zum wahrnehmbaren Übertragen eines Signals auf den Körper des Benutzers und eine Steuereinheit zum periodischen Ansteuern der Signaleinheit 5, 6 mittels eines Ansteuersignals aufweist, wobei eine Periode des Ansteuersignals eine Einatmungsphase und eine Ausatmungsphase umfasst, und wobei das Signal ein Vibrationssignal, ein Stromimpulssignal und/oder ein Temperatursignal ist, und dass das Ansteuersignal unabhängig von physiologischen Körperwerten gebildet ist.

Gemäß einer vorteilhaften Weiterbildung der Erfindung ist vorgesehen, dass die Periode eine Pausenphase umfasst.

Gemäß einer vorteilhaften Weiterbildung der Erfindung ist ein Befestigungsmittel 2 zum Befestigen des Atemtakters 1 am Körper oder an einem körpernahen Kleidungsstück eines Benutzers vorgesehen.

Gemäß einer vorteilhaften Weiterbildung der Erfindung ist vorgesehen, dass das Befestigungsmittel 2 eine Ohrklammer, ein Ohreinsatz, ein Brustband, ein Armband, ein Fußband, eine Klettvorrichtung oder ein Klebestreifen ist.

Gemäß einer vorteilhaften Weiterbildung der Erfindung ist vorgesehen, dass das Signal zusätzlich ein akustisches und/oder optisches Signal umfasst.

Gemäß einer vorteilhaften Weiterbildung der Erfindung ist vorgesehen, dass die Periodendauer, die Dauer der Einatmungsphase, die Dauer der Ausatmungsphase und die Dauer der wenigstens einen optionalen Pausenphase einstellbar sind.

Gemäß einer vorteilhaften Weiterbildung der Erfindung ist vorgesehen, dass die Einatmungsphase eine Dauer von 1 bis 10 Sekunden und die Ausatmungsphase eine Dauer von 2 bis 20 Sekunden aufweist.

Gemäß einer vorteilhaften Weiterbildung der Erfindung ist vorgesehen, dass die Signaleinheit einen Lautsprecher und/oder ein Vibrationselement 5 aufweist.

Gemäß einer vorteilhaften Weiterbildung der Erfindung ist vorgesehen, dass das Signal während der Einatmungsphase eine Vibration zunehmender Intensität und/oder zunehmender Frequenz aufweist.

Gemäß einer vorteilhaften Weiterbildung der Erfindung ist vorgesehen, dass das Signal während der Ausatmungsphase eine Vibration abnehmender Intensität und/oder abnehmender Frequenz aufweist.

Gemäß einer vorteilhaften Weiterbildung der Erfindung ist vorgesehen, dass das Signal während der Einatmungsphase eine sich räumlich fortpflanzende oder ausbreitende Vibration und während der Ausatmungsphase eine sich entgegengesetzt zu der Einatmungsphase verhaltende Vibration aufweist.

Gemäß einer vorteilhaften Weiterbildung der Erfindung ist vorgesehen, dass das Signal während der Einatmungsphase eine zunehmende Temperatur und während der Ausatmungsphase eine abnehmende Temperatur aufweist.

Gemäß einer vorteilhaften Weiterbildung der Erfindung ist vorgesehen, dass die Steuereinheit eingerichtet ist, zu überwachen, ob eine vorgegebene Benutzungsdauer des tragbaren Atemtakters 1 in einer vorgegebenen Zeitspanne erreicht wird.

Gemäß einer vorteilhaften Weiterbildung der Erfindung ist vorgesehen, dass der tragbare Atemtakter 1 wasserdicht ist.

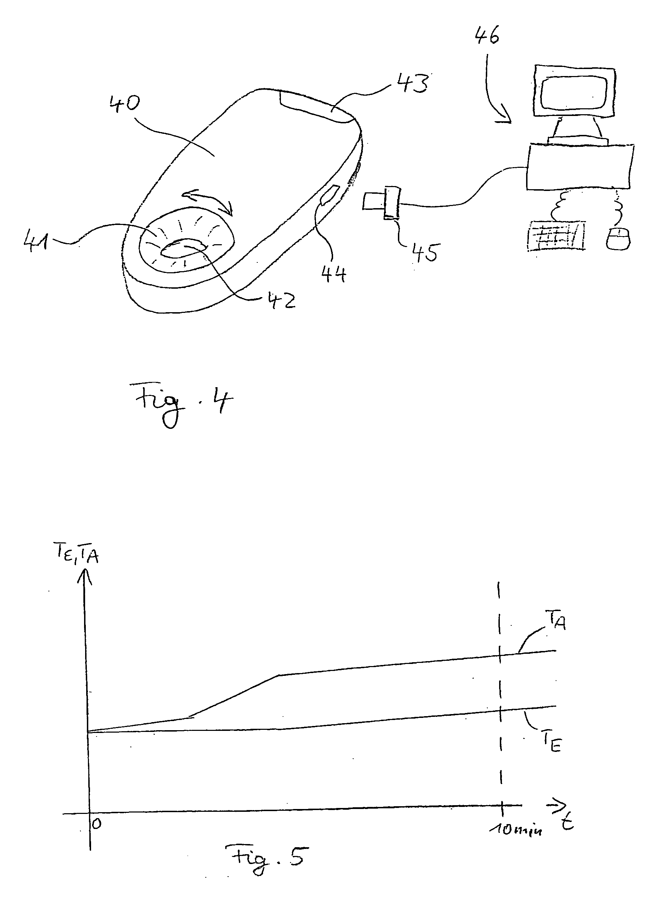

Die Figur 4 zeigt eine weitere Ausführungsform in Form eines Atemtakters 40. Der Atemtakter 40 weist ein Gehäuse auf, an dem von außen zugänglich als Bedienelemente ein Taster 43 sowie ein Drehwähler 41 vorgesehen sind. Der Taster 43 dient beispielsweise zum Ein- und Ausschalten den Atemtakters 40. Mittels des Drehwählers 41 können verschiedene Einstellungen des Atemtakters vorgenommen werden. Beispielsweise können damit verschiedene Atemrhythmen oder Werte für die Benutzungsdauer eingestellt werden.

Der Drehwähler 41 ist in einem dem Taster 43 gegenüberliegenden Bereich des Gehäuses des Atemtakters 40 angeordnet. Der Drehwähler 41 weist ein zu einer Innenseite des Atemtakers 40 hin ringförmig abgerundetes Wählrad auf, das in der Mitte eine Öffnung 42 aufweist. Der Drehwähler 41 ist im Uhrzeigersinn und entgegen des Uhrzeigersinns manuell drehbar. In ergonomisch günstiger Weise kann ein Benutzer mit einem Finger in die Öffnung 42 fassen und hiermit das Wählrad drehen. Das Wählrad ist im Inneren des Gehäuses des Atemtakters 40 beispielsweise mit einer Nockenstruktur versehen. Die Nocken dieser Nockenstruktur stehen in Wirkverbindung mit elektrischen Kontakten, sodass beim Drehen des Drehwählers 41 jeweils unterschiedliche elektrische Kontakte geschlossen bzw. geöffnet werden. Hierdurch kann eine in dem Atemtakter 40 vorgesehene Auswerteeinheit die Einstellwünsche eines Benutzers erfassen und entsprechend die gewünschten Atemrhythmen und Perioden ausgeben.

Der Atemtakter 40 weist ferner einen Schnittstellenanschluss 44 auf, der zum Anschluss eines zugeordneten Steckverbinders 45 dient. Über den Steckverbinder 45 kann ein Computer 46 mit dem Atemtakter 40 verbunden werden. Der Anschluss 44 kann auch als drahtlose Schnittstelle, beispielsweise als Funkschnittstelle oder Infrarotschnittstelle, ausgestaltet sein. Über den Computer 46 können Einstellungen an dem Atemtakter 40 vorgenommen werden und von dem Atemtakter 40 aufgezeichnete Werte, wie z. B. Blutdruck und Pulsfrequenz des Benutzers sowie die Benutzungsdauer des Atemtakters, ausgelesen werden.

In der Figur 5 ist beispielhaft eine automatische Veränderung von Atemrhythmen während einer Benutzungsperiode des Atemtakters dargestellt. Ausgehend von einem Benutzungsbeginn zum Zeitpunkt t=0 gibt der Atemtakter Signale aus, durch die eine relativ geringe Dauer der Einatmungsphase T_{E} und eine gleiche oder etwas längere Dauer der Ausatmungsphase T_{A} vorgegeben werden. Im Laufe der Benutzung wird zu bestimmten Zeitpunkten der Wert für die Dauer der Einatmungsphase langsam erhöht. Entsprechend wird auch während der Benutzungsdauer die Dauer der Ausatmungsphase langsam erhöht, und zwar in dem Beispiel gemäß Figur 5 etwas frühzeitiger als die Erhöhung der Dauer der Einatmungsphase. Beispielsweise kann mit gleichen Zeiten von z. B. 4 Sekunden für die Dauer der Einatmungsphase und die Dauer der Ausatmungsphase begonnen werden. Nach einem gewissen Zeitraum wird die Dauer der Ausatmungsphase z. B. auf 6 Sekunden erhöht. Zu einem weiteren, späteren Zeitpunkt kann beispielsweise die Dauer der Einatmungsphase auf 5 Sekunden und die Dauer der Ausatmungsphase auf 8 Sekunden erhöht werden. Eine solche automatische Veränderung des Atemrhythmus kann sich, wie in der Figur 5 dargestellt, beispielsweise über eine Benutzungsperiode von 10 Minuten erstrecken.

## Patentansprüche

1. Tragbarer Atemtakter mit einer Signaleinheit (5, 6) zum wahrnehmbaren Übertragen eines Signals auf den Körper eines Benutzers und mit einer Steuereinheit zum periodischen Ansteuern der Signaleinheit (5, 6) mittels eines Ansteuersignals, wobei eine Periode des Ansteuersignals wenigstens eine Einatmungsphase und eine Ausatmungsphase umfasst, wobei die Steuereinheit dazu eingerichtet ist, die Dauer der Einatmungsphase (T_{E}) und/oder der Ausatmungsphase (T_{A}) während einer Benutzungsperiode des Atemtakters automatisch zu verändern, **dadurch gekennzeichnet, dass** ein Bedienelement (15, 16, 41, 43) vorgesehen ist, das nur bestimmte voreingestellte Kombinationen der Dauer der Einatmungsphase (T_{E}) und der Ausatmungsphase (T_{A})erlaubt, wobei die Ausatmungsphase (T_{A}) gleich oder länger als die Einatmungsphase (T_{E}) ist.

2. Atemtakter nach Anspruch 1, **dadurch gekennzeichnet, dass** die Steuereinheit dazu eingerichtet ist, die Dauer der Einatmungsphase (T_{E}) und/oder der Ausatmungsphase (T_{A}) während einer Benutzungsperiode des Atemtakters zu größeren Werten hin zu verändern.

3. Atemtakter nach wenigstens einem der vorhergehenden Ansprüche, **dadurch gekennzeichnet, dass** die Steuereinheit zur Abfrage des Bedienelements (15, 16, 41, 43) eingerichtet ist, wobei über das Bedienelement (15, 16, 41, 43) die Dauer der Einatmungsphase (T_{E}) und/oder die Dauer der Ausatmungsphase (T_{A}) in Abhängigkeit von der Körpergröße und dem Körpergewicht eines Benutzers einstellbar ist.

4. Atemtakter nach wenigstens einem der vorhergehenden Ansprüche, **dadurch gekennzeichnet, dass** die Steuereinheit Mittel aufweist, die eine Einstellung einer Einatmungsphase (T_{E}) auf eine längere Dauer als die Dauer der Ausatmungsphase (T_{A}) verhindern.

5. Atemtakter nach wenigstens einem der vorhergehenden Ansprüche, **dadurch gekennzeichnet, dass** der Atemtakter mit einem Wirtsgerät baulich vereint oder damit integriert ausgebildet ist.

6. Atemtakter nach wenigstens einem der vorhergehenden Ansprüche, **gekennzeichnet durch** eine Ausgestaltung als Computerprogrammprodukt.

7. Verwendung eines Atemtakters nach wenigstens einem der vorhergehenden Ansprüche mit einer Mehrzahl von Benutzern, wobei in dem Atemtakter gespeicherte Werte ausgelesen und in einer Benutzerdatenbank gespeichert werden und durch eine eindeutige Geräte-Identifikation des Atemtakters einem Benutzer aus einer Vielzahl von in der Benutzerdatenbank verwalteten Benutzern zugeordnet wird.

8. Verwendung eines Atemtakters nach Anspruch 7, **dadurch gekennzeichnet, dass** die ausgelesenen Werte einen bevorzugten Atemrhythmus und/oder eine bevorzugte Benutzungsdauer durch einen Benutzer beinhalten.

9. Verwendung eines Atemtakters nach Anspruch 7 oder 8, **dadurch gekennzeichnet, dass** die ausgelesenen Werte wenigstens einen Körperzustand des Benutzers beinhalten.

## Claims

1. Portable breath regulator with a signal unit (5, 6) for the perceptible transmission of a signal to the body of a user, and with a control unit for periodically actuating the signal unit (5, 6) by means of an actuation signal, wherein a period of the actuation signal comprises at least one inhalation phase and one exhalation phase, wherein the control unit is designed to automatically modify the duration of the inhalation phase (T_{E}) and/or of the exhalation phase (T_{A}) during a period of use of the breath regulator, **characterized in that** an operating element (15, 16, 41, 43) is provided, which allows only specific pre-set combinations of the duration of the inhalation phase (T_{E}) and of the exhalation phase (T_{A}), wherein the exhalation phase (T_{A}) is the same as or longer than the inhalation phase (T_{E}).

2. Breath regulator according to Claim 1, **characterized in that** the control unit is designed to modify the duration of the inhalation phase (T_{E}) and/or of the exhalation phase (T_{A}), during a period of use of the breath regulator, towards higher values.

3. Breath regulator according to at least one of the preceding claims, **characterized in that** the control unit is designed to interrogate the operating element (15, 16, 41, 43), wherein the duration of the inhalation phase (T_{E}) and/or the duration of the exhalation phase (T_{A}) can be adjusted, via the operating element (15, 16, 41, 43), depending on the size and weight of a user.

4. Breath regulator according to at least one of the preceding claims, **characterized in that** the control unit has means that prevent adjustment of an inhalation phase (T_{E}) to a duration longer than the duration of the exhalation phase (T_{A}).

5. Breath regulator according to at least one of the preceding claims, **characterized in that** the breath regulator is structurally combined with a host device or integrated therein.

6. Breath regulator according to at least one of the preceding claims, **characterized by** a configuration as a computer program product.

7. Use of a breath regulator according to at least one of the preceding claims with a plurality of users, wherein values stored in the breath regulator are read out and stored in a user database and, by unambiguous device identification of the breath regulator, are assigned to a user from a large number of users managed in the user database.

8. Use of a breath regulator according to Claim 7, **characterized in that** the read-out values contain a preferred breathing rhythm and/or a preferred duration of use by a user.

9. Use of a breath regulator according to Claim 7 or 8, **characterized in that** the read-out values contain at least one physical state of the user.

## Revendications

1. Régulateur de cycle respiratoire portatif comprenant une unité à signaux (5, 6) pour transmettre de façon perceptible un signal au corps d'un utilisateur, et comprenant une unité de commande pour piloter périodiquement l'unité à signaux (5, 6) au moyen d'un signal de pilotage, dans lequel une période du signal de pilotage inclut au moins une phase d'inspiration et une phase d'expiration, dans lequel l'unité de commande est conçue pour modifier automatiquement la durée de la phase d'inspiration (TE) et/ou de la phase d'expiration (TA) pendant une période d'utilisation du régulateur de cycle respiratoire, **caractérisé en ce qu'**il est prévu un élément de manipulation (15, 16, 41, 42) qui ne permet que certaines combinaisons préréglées de durée de la phase d'inspiration (TE) et de la phase d'expiration (TA), et dans lequel la phase d'expiration (TA) est égale à la phase d'inspiration (TE) ou plus longue que celle-ci.

2. Régulateur de cycle respiratoire selon la revendication 1, **caractérisé en ce que** l'unité de commande est conçue pour modifier la durée de la phase d'inspiration (TE) et/ou de la phase d'expiration (TA) pendant une période d'utilisation du régulateur de cycle respiratoire en allant vers des valeurs plus élevées.

3. Régulateur de cycle respiratoire selon l'une au moins des revendications précédentes, **caractérisé en ce que** l'unité de commande est conçue pour interroger l'élément de manipulation (15, 16, 41, 43), et dans lequel la durée de la phase d'inspiration (TE) et/ou la durée de la phase d'expiration (TA) est réglable via l'élément de manipulation (15, 16, 41, 43) en fonction de la taille corporelle et du poids corporel d'un utilisateur.

4. Régulateur de cycle respiratoire selon l'une au moins des revendications précédentes, **caractérisé en ce que** l'unité de commande comprend des moyens qui empêchent d'établir une phase d'inspiration (TE) à une durée plus longue que la durée de la phase d'expiration (TA).

5. Régulateur de cycle respiratoire selon l'une au moins des revendications précédentes, **caractérisé en ce que** le régulateur de cycle respiratoire est réalisé avec un appareil hôte, soit réuni sur le plan structurel soit intégré avec celui-ci.

6. Régulateur de cycle respiratoire selon l'une au moins des revendications précédentes, **caractérisé par** un mode de réalisation à titre de produit de programme d'ordinateur.

7. Utilisation d'un régulateur de cycle respiratoire selon l'une au moins des revendications précédentes avec une pluralité d'utilisateurs, dans laquelle des valeurs mémorisées dans le régulateur de cycle respiratoire sont lues et mémorisées dans une base de données utilisateur, et sont associées à un utilisateur, par une identification d'appareil univoque du régulateur de cycle respiratoire parmi une pluralité d'utilisateurs gérés dans la base de données utilisateur.

8. Utilisation d'un régulateur de cycle respiratoire selon la revendication 7, **caractérisé en ce que** les valeurs lues incluent un rythme respiratoire préféré et/ou une durée d'utilisation préférée par un utilisateur.

9. Utilisation d'un régulateur de cycle respiratoire selon la revendication 7 ou 8, **caractérisé en ce que** les valeurs lues incluent au moins un état corporel de l'utilisateur.
